(19) Europäisches Patentamt
European Patent Office
Office européen des brevets

(11) Numéro de publication : **0 374 029 B1**

# (12) FASCICULE DE BREVET EUROPEEN

(45) Date de publication du fascicule du brevet : **25.03.92 Bulletin 92/13**

(51) Int. Cl.$^5$ : **A61K 31/415,** A61K 31/42, A61K 31/425

(21) Numéro de dépôt : **89403431.3**

(22) Date de dépôt : **12.12.89**

## (54) Application de benzamides substitués comme gastromoteurs.

(30) Priorité : **14.12.88 FR 8816433**

(43) Date de publication de la demande : **20.06.90 Bulletin 90/25**

(45) Mention de la délivrance du brevet : **25.03.92 Bulletin 92/13**

(84) Etats contractants désignés : **AT BE CH DE ES FR GB GR IT LI LU NL SE**

(56) Documents cités :
**EP-A- 0 236 646**
**WO-A-86/02553**
**US-A- 4 188 393**
**US-A- 4 714 707**

(73) Titulaire : **Laboratoires DELAGRANGE**
**1, Avenue Pierre-Brossolette**
**F-91380 Chilly-Mazarin (FR)**

(72) Inventeur : **Acher, Jacques**
**32, route de Saint Vrain**
**F-91760 Itteville (FR)**
Inventeur : **Monier, Jean-Claude**
**6, rue de la Sorbonne**
**F-91510 Lardy (FR)**
Inventeur : **Schmitt, Jean-Paul**
**13, rue Victor Hugo**
**F-91290 Arpajon (FR)**
Inventeur : **Gardaix-Luthereau, Renée**
**9, rue Guichard**
**F-94230 Cachan (FR)**
Inventeur : **Costall, Brenda Dr.**
**The Old Rectory Addingham**
**Ilkey West Yorkshire (GB)**
Inventeur : **Naylor, Robert Dr.**
**The Old Rectory Addingham**
**Ilkey West Yorkshire (GB)**

**EP 0 374 029 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une nouvelle application de benzamides substitués de formule (I) :

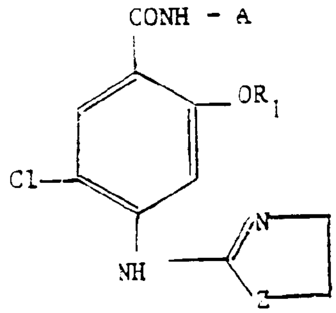

(I)

dans laquelle :

- A représente un groupe $C_1$-$C_3$ alkyle linéaire ou ramifié, un groupe allyle ou diéthylamino éthyle,
- $R_1$ représente un atome d'hydrogène ou un groupe méthyle,
- Z représente un groupe NH, un atome d'oxygène ou de soufre,

et de leurs sels pharmacologiquement acceptables.

En particulier, l'invention concerne les composés suivants :

Composé I : N-[2-(diéthylamino) éthyl] 2-méthoxy 4-[(1H-4,5-dihydro 2-imidazolyl) amino] 5- chloro benzamide.

Composé II : N-[2-(diéthylamino) éthyl] 2-méthoxy 4-[(4,5-dihydro 2-thiazolyl)amino] 5-chloro benzamide.

Composé III : N-[2-(diéthylamino)éthyl] 2-méthoxy 4-[(4,5-dihydro 2-oxazolyl)amino] 5-chloro benzamide.

Composé IV : N-(méthyl) 2-méthoxy 4-[(1H-4,5-dihydro 2-imidazolyl) amino] 5-chloro benzamide.

Composé V : N- 2-(diéthylamino) éthyl 2-hydroxy 4- (1H-4,5-dihydro 2-imidazolyl)amino 5-chlorobenzamide

Composé VI : N-(éthyl) 2-méthoxy 4- (1H-4,5-dihydro 2-imidazolyl) amino 5-chlorobenzamide.

Composé VII : N-(isopropyl) 2-méthoxy 4- (1H-4,5-dihydro 2-imidazolyl) amino 5-chloro benzamide.

Composé VIII: N-(allyl) 2-méthoxy 4- (1H-4,5-dihydro 2-imidazolyl) amino 5-chloro benzamide.

Ces composés sont inclus dans la formule générale de la demande de brevet français FR-A-2 592 042 (=EP-A-0 236 646).

La préparation des composés I à III, VII et VIII a été spécifiquement décrite dans cette demande de brevet.

Les composés IV à VI ont été préparés selon un procédé analogue, de la façon suivante :

<u>Préparation du N-(méthyl) 2-méthoxy 4- (1H,4,5-dihydro 2-imidazolyl) amino 5-chlorobenzamide (composé IV)</u>

Dans un tricol de 500ml, on a introduit 150ml de méthanol et 2,3g de sodium. Lorsque tout le sodium a été consommé, on a ajouté 26,95g d'acide 2-méthoxy 4- (1H-4,5-dihydro 2-imidazolyl) amino 5-chloro benzoïque. Il y a eu dissolution puis cristallisation. Le solvant a été chassé à sec sous vide puis les cristaux ont été broyés et chargés dans un tricol de 500ml avec 100ml de chloroforme. On a versé lentement sur la suspension, 22ml de chlorure de thionyle. Il y a eu un dégagement gazeux et la température est montée à 30°C. On a chauffé 2 heures à 50°C puis refroidi et chassé le solvant. Le résidu a été versé dans 150ml d'une solution aqueuse de méthylamine à 30%. Après une heure d'agitation, on a essoré, lavé à l'eau et séché à 70°C.

On a obtenu 20,81g de produit (rendement=73,7%).18,89 g de produit ont été dissous dans 180ml d'eau et 10ml d'acide acétique, traités au noir, filtrés, précipités par 25ml d'ammoniaque, essorés, lavés et séchés à 70°C. On a obtenu 17,5g de produit. Par RMN on a détecté une impureté estimée à 3% et des traces d'ester. 16,5 g de produit ont été dissous à reflux dans 215 ml de diméthylformamide, traités au noir, filtrés à chaud, cristallisés à froid, essorés, lavés au diméthylformamide, puis à l'eau et séchés. On a obtenu 13g de produit, contenant encore une impureté. Ces 13g ont été redissous dans 130ml d'eau et 8ml d'acide acétique. La solution a été filtrée puis le précipité, obtenu par addition de 20ml d'ammoniaque, a été essoré, lavé et séché à 70°C. On a obtenu 12,5g de produit (rendement total=51,7%)

P.F. supérieur à 260°C.

| | | | |
|---|---|---|---|
| Analyse : | $H_2O$ | : 0,5% | |
| | titre (corrigé) | : 98,8% | |
| | Cl (corrigé) | : 12,63% | (calculé : 12,54%) |
| | N (corrigé) | : 19,57% | (calculé : 19,82%) |

Les spectres RMN et IR étaient compatibles avec la structure attendue.

- Préparation du N-[2-(diéthylamino)éthyl] 2-hydroxy 4-[(1H-4,5-dihydro 2- imidazolyl) amino] 5-chloro benzamide (composé V)

Stade 1 : chlorhydrate de N-[2-(diéthylamino) éthyl] 2- hydroxy 4-isothiocyanato 5-chloro benzamide.

Dans un tricol de 1 litre équipé d'un agitateur, d'un thermomètre, d'un réfrigérant et d'une ampoule d'introduction, on a chargé 57g de N-[2-(diéthylamino)éthyl] 2-hydroxy 4-amino 5-chloro benzamide et 300 ml de tétrachlorure de carbone puis ajouté une solution de 20ml de thiophosgène dans 100ml de tétrachlorure de carbone.

On a porté 3 heures au reflux sous agitation puis ajouté une solution de 8ml de thiophosgène dans 50ml de tétrachlorure de carbone.

On a laissé encore 4 heures au reflux, puis refroidi à 15°C, essoré, lavé deux fois avec 50ml de tétrachlorure de carbone puis trois fois avec 50ml d'acétone et séché.

On a obtenu 68, 63g de produit (rendement = 94%) P.F. = 153°C.

Stade 2 : N-[2-(diéthylamino)éthyl] 2-hydroxy 4-[N'-(2-aminoéthyl) thiouréido]5-chloro benzamide

Dans un bécher, on a chargé sous agitation, 36,4g de chlorhydrate de N- 2-(diéthylamino)éthyl 2-hydroxy 4-isothiocyanato 5-chloro benzamide, 200ml de chlorure de méthylène et 10g de triéthylamine. On a agité 15 minutes puis filtré les sels formés.

Le filtrat a été versé sous agitation, en 40 minutes, dans un tricol contenant 15g d'éthylènediamine et 200ml d'éther isopropylique, en maintenant la température inférieure à - 10°C.

On a agité 3 heures en laissant remonter la température puis filtré le précipité. Ce précipité a été rechargé dans le ballon. On a ajouté 200ml d'eau, agité 1 heure, essoré, lavé à l'eau et séché à 40°C. On a obtenu 34,52g de produit (rendement=89%) P.F.= décomposition instantanée vers 160-170°C, puis la température de fusion décroît.

Stade 3 : N-[2-(diéthylamino) éthyl] 2-hydroxy 4-[(1H-4,5-dihydro 2-imidazolyl)amino] 5-chloro benzamide.

Dans un tricol de 1 litre équipé d'un agitateur, d'un thermomètre et d'un réfrigérant, on a chargé 62g de N-[2-(diéthylamino)éthyl]2-hydroxy 4-[N'-(2-aminoéthyl)thiouréido]5-chloro benzamide et 500ml de carbonate d'éthyle.

On a porté 2 heures à reflux, dans un bain d'huile à 155-160°C. On a ajouté du noir végétal, laissé encore au reflux pendant 10 minutes, filtré à chaud et lavé le filtre deux fois avec 50ml de carbonate d'éthyle chaud. Le produit a été recueilli dans un bécher sous agitation. Au début du refroidissement, une gomme grise a adhéré aux parois. On a transvasé la masse encore chaude dans une autre bécher et refroidi à -10°C, sous agitation.

Le précipité a été essoré, lavé et empâté par 4x50ml d'éther de pétrole puis séché. On a obtenu 25,25g de produit contenant des impuretés.

24,8g de produit et 225ml d'acétate de butyle ont été portés à reflux 10 minutes. On a refroidi à 0-5°C, essoré, lavé et empâté par 4x50ml d'éther isopropylique puis séché.

On a obtenu 17,5g de produit contenant encore une impureté. 17g de produit ont été repris à reflux dans 85ml d'acétate de butyle, refroidis à 5°C, essorés, lavés et empâtés par 4x40ml d'éther isopropylique puis séchés.

On a obtenu 15,32g de produit. On a dissous à reflux, 9,6g de produit dans 300ml de carbonate d'éthyle. La solution a été traitée au noir puis filtrée. Les cristaux formés à froid ont été essorés, lavés à l'éther éthylique et séchés à 60°C sous vide puis 72 heures à 80°C, sous vide.

On a obtenu 6g de produit (rendement total = 17,7%) P.F. = 151°C, recristallise et fond à 205°C.

Analyse : Cl : 10,27% (calculé : 10,02%)
titre : 96%

Le spectre RMN était compatible avec la structure attendue.

- Préparation du N-(éthyl) 2-méthoxy 4-[(1H-4,5- dihydro 2-imidazolyl) amino] 5-chloro benzamide (composé VI).

Dans un tricol de 500ml, équipé d'un agitateur, d'un thermomètre et d'un réfrigérant, on a introduit 56,7g de 2-méthoxy 4-[(1H-4,5-dihydro 2-imidazolyl) amino] 5-chloro benzoate de méthyle, 100 ml d'éthylène glycol et 200 ml d'une solution aqueuse d'éthylamine à 60%. On a chauffé 120 heures à 75°C sous agitation, ajouté 10 ml de lessive de soude puis agité encore une heure à 75°C et refroidi.
Le précipité a été essoré, lavé à l'eau et séché.
On a obtenu 42,5g de produit (rendement : 72%). Ces 42,5g de produit ont été recristallisés dans 512 ml de méthanol. Le précipité a été essoré, lavé au méthanol et séché à 75-80°C.
On a obtenu 38,2 g de produit, qui ont été redissous dans 400 ml d'eau et de l'acide acétique. On a filtré la solution, précipité le produit par de l'ammoniaque et laissé cristalliser une nuit au réfrigérateur. Les cristaux ont été essorés, lavés à l'eau et séchés à 70°C sous vide, sur anhydride phosphorique.
On a obtenu 34,5 g de produit (rendement de la purification : 80,7%) - P.F. = 237°C.

Les spectres RMN et IR étaient compatibles avec la structure attendue.

Les composés de formule (I), faisant l'objet d'études nouvelles et approfondies, ont révélé, outre leur action sur le système nerveux central, une très puissante activité sur la motricité gastrique, ce qui n'était pas prévisible d'après les propriétés qu'on leur connaissait jusqu'alors.

Une telle activé présente en particulier l'intérêt de conduire à de nouveaux médicament améliorant la gastromotricité, la posologie étant fixée par le médecin traitant en fonction de l'atteinte.

Des tests pharmacologiques démontrent l'activité précitée, et son intérêt.

Afin de comparer l'action des composés selon l'invention à celle du METOCLOPRAMIDE, produit de référence dans ce domaine, l'étude "in vivo" chez le cobaye a été menée selon le protocole suivant :

La nourriture des animaux a été supprimée 14 heures avant la mesure de la vidange gastrique. L'expérimentation a été réalisée sous faible éclairage, avec le moins possible de bruits et de perturbations, uniquement par des expérimentateurs ayant un contact quotidien avec les cochons d'inde, et ayant conduit un entraînement initial pour habituer les animaux à la manipulation. Ces animaux ont donc été soumis à un stress minimal.

La mesure de la vidange gastrique a été assurée par localisation aux rayons-X (50 KV, 30mA, 0,5-0,8s.), sur plaques Kodak (NS-2T, 13x18cm), de sphéroïdes de sulfate de baryum (environ 30, de 1 mm de diamètre) revêtus de polystyrène, qu'on avait fait avaler aux cochons d'inde en les plaçant au fond de la gorge, dans une solution de 0,2ml de carboxymethylcellulose à 1%, avec 0,05ml de glycérine, de façon à initier une déglutition rapide et volontaire.

Le passage des sphéroïdes a été suivi pendant 3 à 4 heures, les animaux étant placés dans leurs cages habituelles pendant ce temps, et sortis 30-60 minutes). Durant la procédure aux rayons-X, ils ont été placés dans des cages individuelles de contention, les maintenant confortablement dans une position stable, la cage étant correctement façonnée (33 x 15cm et 13cm de haut) pour maintenir un cochon d'inde de 450 à 550g entre les parois capitonnées et l'animal étant entraîné, à entrer dans la cage pour y rester tranquille et non stressé.

Schéma expérimental

La vidange gastrique a été mesurée par le nombre de sphéroïdes sortant de l'estomac. Pour chaque dose du produit étudié, 6 cochons d'inde ont été utilisés, et les réponses ont été comparées à celles de cochons d'inde recevant le véhicule approprié.

Le composé (I) a été étudié aux doses de 1mg/kg et 0,01mg/kg, les composés (II) à (V) à la dose de 1mg/kg, les composés (VI) à (VIII) à la dose de 5mg/kg et le METOCLOPRAMIDE aux doses de 1mg/kg et 5mg/kg.

Les résultats sont consignés dans la figure 1, sous forme graphique.

Les composés selon l'invention et le METOCLOPRAMIDE ont été dissous dans de l'eau distillée et les doses sont exprimées en base.

Résultats de l'étude "in vivo"

L'administration i.p de METOCLOPRAMIDE et des composés selon l'invention provoque une augmentation de la vidange gastrique (voir figure 1) chez le cobaye immobilisé.

Cette vidange est exprimée, dans la figure 1 et le tableau 1, en pourcentage de sphéroïdes quittant l'estomac.

Vidange gastrique en fonction du temps, chez le cobaye, après administration I.P. de 1mg/kg de composé.

| Composé | après 1 heure | après 2 heures |
|---|---|---|
| I | 24 | 63 |
| II | 12,5 | 60 |
| III | 20 | 57 |
| IV | 38 | 62 |
| V | 32 | 55 |
| METOCLOPRAMIDE | 16 | 39 |

TABLEAU 1

D'après les résultats obtenus, il apparaît que la vidange gastrique est augmentée de façon significative par rapport à celle du groupe témoin : Les composés (I) à (V) administrés à la dose de 1mg/kg ont provoqué une vidange gastrique de 55 à 63% au bout de 2 heures alors que celle du groupe témoin était de 28%. Les composés (VII) et (VIII) administrés à la dose de 5mg/kg ont provoqué une vidange gastrique de 46,2 et 47,4% respectivement au bout de 2 heures alors que celle du groupe témoin était de 24,8%. Il apparaît également que les composés selon l'invention sont plus puissants que le METOCLOPRAMIDE : on constate par exemple, qu'à raison de 1mg/kg des composés (I) et (IV) à l'étude, on obtient un résultat équivalent à l'emploi de 5mg/kg du produit de référence. Il y a donc bien une action augmentant la gastromotricité.

D'autre part, la toxicité aiguë par voie intraveineuse de composé de l'invention a été étudiée chez la souris mâle.

Les doses léthales 50 ($DL_{50}$) suivantes ont ainsi été déterminées :

| Composé | DL 50 en mg/kg |
|---|---|
| I | 12,8 - 14,4 |
| II | 40,6 - 48,7 |
| III | 91 - 116 |
| IV | 95,8 - 138 |
| V | 65,7 - 76,7 |
| METOCLOPRAMIDE | 25,4 - 37,9 |

Les composés selon l'invention peuvent être administrés sous forme de comprimés, gélules, sirop ou autres formes galéniques usuelles destinées à l'administration orale ou parentérale, en association avec des excipients solides ou liquides.

## Revendications

1. Utilisation des benzamides substitués de formule (I) :

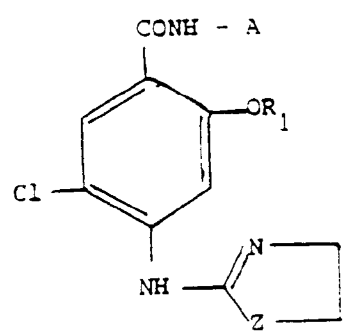

(I)

dans laquelle :

- A représente un groupe $C_1$-$C_3$ alkyle linéaire ou ramifié, un groupe allyle ou diéthylaminoéthyle,
- $R_1$ représente un atome d'hydrogène ou un groupe méthyle,
- Z représente un groupe NH, un atome d'oxygène ou de soufre,

et de leurs sels d'addition pharmacologiquement acceptables, pour la préparation de médicaments actifs comme gastromoteurs.

2. Utilisation selon la revendication 1, du N-[2-(diéthylamino) éthyl] 2-méthoxy 4-[1H-4,5-dihydro 2-imidazolyl)amino] 5-chlorobenzamide.

3. Utilisation selon la revendication 1, du N-[2-diéthylamino) éthyl] 2-méthoxy 4-[(4,5-dihydro 2-thiazolyl)amino] 5-chloro benzamide.

4. Utilisation selon la revendication 1, du N-[2-(diéthylamino)éthyl] 2-méthoxy 4-[(4,5-dihydro 2-oxazolyl) amino] 5-chloro benzamide.

5. Utilisation selon la revendication 1, du N-(méthyl) 2-méthoxy 4-[(1H-4,5-dihydro 2-imidazolyl) amino] 5-chloro benzamide.

6. Utilisation selon la revendication 1, du N-[2-(diéthylamino) éthyl] 2-hydroxy 4-[(1H-4,5-dihydro 2-imidazolyl)amino] 5-chloro benzamide.

7. Utilisation selon la revendication 1, du N-(ethyl) 2-méthoxy 4-[(1H-4,5-dihydro 2-imidazolyl) amino] 5-chloro benzamide.

8. Utilisation selon la revendication 1, du N-(isopropyl) 2-méthoxy 4-[(1H-4,5-dihydro 2-imidazolyl) amino] 5-chloro benzamide.

9. Utilisation selon la revendication 1, du N-(allyl) 2-méthoxy 4-[(1H-4,5-dihydro 2-imidazolyl) amino] 5-chloro benzamide.

## Patentansprüche

1. Verwendung von substituierten Benzamiden der Formel (I)

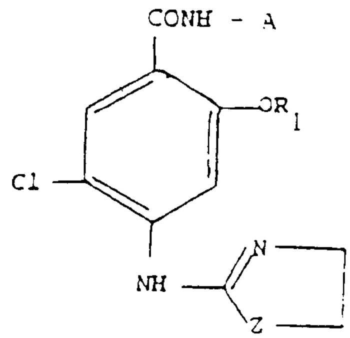

(I)

in der

– A eine lineare oder verzweigte $C_1$-$C_3$-Alkylgruppe, eine Allylgruppe oder eine Diethylaminoethylgruppe,
– $R_1$ ein Wasserstoffatom oder eine Methylgruppe und
– Z eine NH-Gruppe, ein Sauerstoffatom oder ein Schwefelatom

bedeuten, sowie von pharmakologisch verträglichen Säureadditionssalzen derselben, zur Herstellung von als gastromotorische Mittel wirksamen Medikamenten.

2. Verwendung nach Anspruch 1 von N-[2-(Diethylamino)ethyl]-2-methoxy-4-[(1H-4,5-dihydro-2-imidazolyl)amino]-5-chlorbenzamid.

3. Verwendung nach Anspruch 1 von N-[2-(Diethylamino)ethyl]-2-methoxy-4-[(4,5-dihydro-2-thiazolyl)amino]-5-chlorbenzamid.

4. Verwendung nach Anspruch 1 von N-[2-(Diethylamino)ethyl]-2-methoxy-4-[(4,5-dihydro-2-oxazolyl)-amino]-5-chlorbenzamid.

5. Verwendung nach Anspruch 1 von N-(Methyl)-2-methoxy-4-[(1H-4,5-dihydro-2-imidazolyl)-amino]-5-chlorbenzamid.

6. Verwendung nach Anspruch 1 von N-[2-(Diethylamino)ethyl]-2-hydroxy-4-[(1H-4,5-dihydro-2-imidazolyl)amino]-5-chlorbenzamid.

7. Verwendung nach Anspruch 1 von N-(Ethyl)-2-methoxy-4-[(1H-4,5-dihydro-2-imidazolyl)amino]-5-chlorbenzamid.

8. Verwendung nach Anspruch 1 von N-(Isopropyl)-2-methoxy-4-[(1H-4,5-dihydro-2-imidazolyl)-amino]-5-chlorbenzamid.

9. Verwendung nach Anspruch 1 von N-(Allyl)-2-methoxy-4-[(1H-4,5-dihydro-2-imidazolyl)amino]-5-chlorbenzamid.

## Claims

1. Use of substituted benzamides of formula (I):

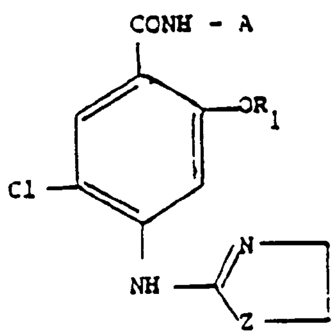

(I)

in which:

– A represents a straight or branched $C_1$-$C_3$ alkyl group, an allyl or diethylaminoethyl group,

– $R_1$ represents a hydrogen atom or a methyl group,

– Z represents a NH, an oxygen or sulphur atom, and their pharmacologically acceptable addition salts, for the preparation of medicaments active as gastromotors.

2. Use according to claim 1 of N-[2-(diethylamino)-ethyl]-2-methoxy-4-[1H-4,5-dihydro-2-imidazolyl)-aminol-5-chlorobenzamide.

3. Use according to claim 1 of N-[2-(diethylamino)-ethyl]-2-methoxy-4-[(4,5-dihydro-2-thiazolyl)-amino]-5-chlorobenzamide.

4. Use according to claim 1 of N-[2-(diethylamino)-ethyl]-2-methoxy-4-[(4,5-dihydro-2-oxazolyl)-amino]-5-chlorobenzamide.

5. Use according to claim 1 of N-(methyl)-2-methoxy-4-[(1H-4,5-dihydro-2-imidazolyl)-amino]-5-chlorobenzamide.

6. Use according to claim 1 of N-[2-(diethylamino)-ethyl]-2-hydroxy-4-[(1H-4,5-dihydro-2-imidazolyl)-amino]-5-chlorobenzamide.

7. Use according to claim 1 of N-(ethyl)-2-methoxy-4-[(1H-4,5-dihydro-2-imidazolyl)-amino]-5-chlorobenzamide.

8. Use according to claim 1 of N-(isopropyl)-2-methoxy-4-[(1H-4,5-dihydro-2-imidazolyl)-amino]-5-chlorobenzamide.

9. Use according to claim 1 of N-(allyl)-2-methoxy-4-[(1H-4,5-dihydro-2-imidazolyl)-amino]-5-chlorobenzamide.

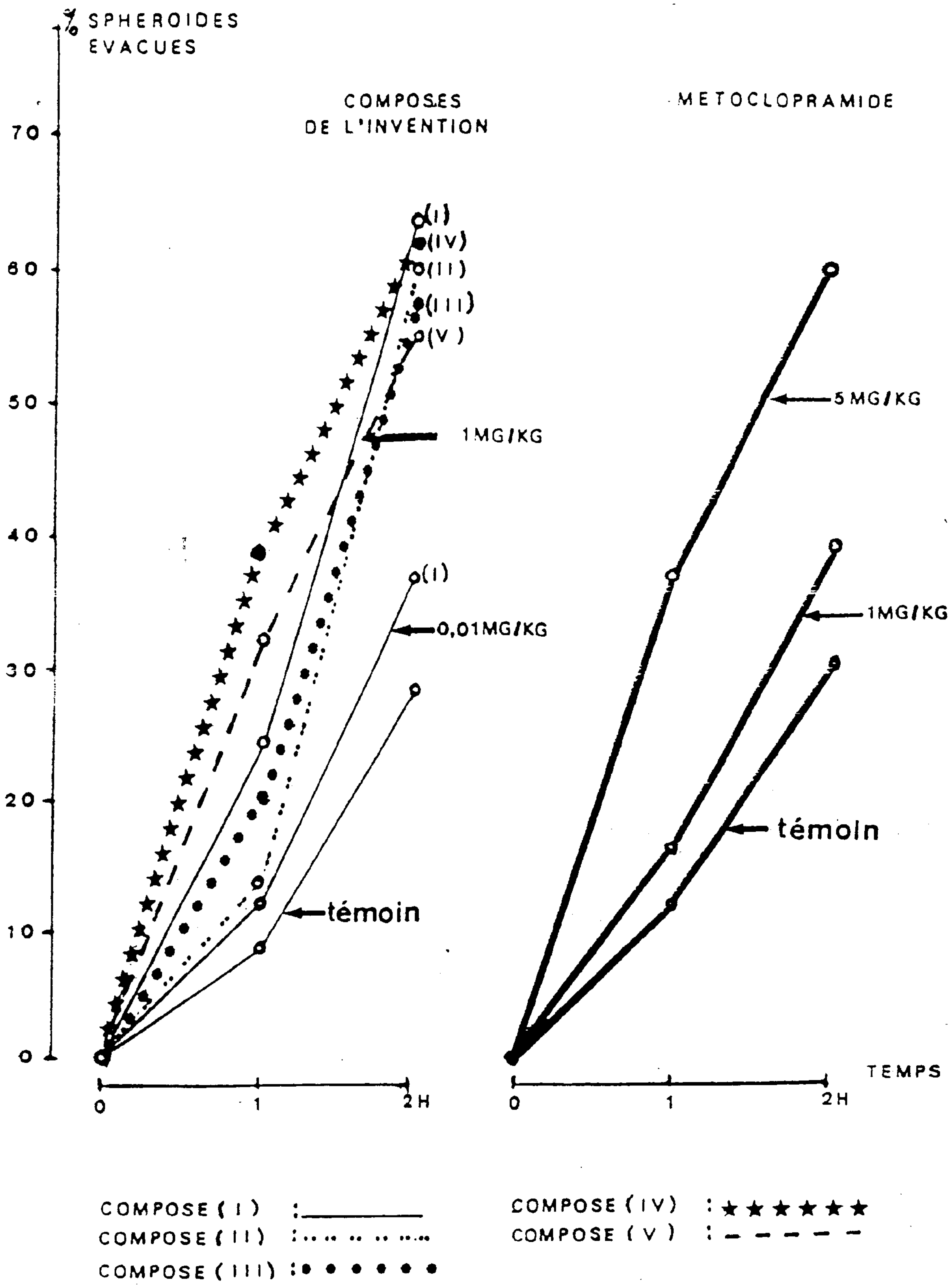


FIGURE 1 : CAPACITE D'AMELIORATION DE LA VIDANGE GASTRIQUE.